# EUROPEAN PATENT APPLICATION

(11) **EP 0 667 398 A2**
(43) Date of publication of application: **16.08.1995**
(21) Application number: 95101441.4
(22) Date of filing: 02.02.1995
(51) Int. Cl.: C12Q 1/68, G01N 21/65, G01N 21/03

(54) **Method of and apparatus for detecting specific base sequence of DNA**

(30) Priority: 14.02.1994 JP 40556/94
(71) Applicant: Kyoto Dai-ichi Kagaku Co., Ltd., Minami-ku, Kyoto 601 (JP)
(72) Inventor: Xiaoming, Dou, c/o Kyoto Dai-ichi Kagaku Co., Ltd., Kyoto 601 (JP); Uenoyama, Harumi, c/o Kyoto Dai-ichi Kagaku Co,Ltd, Kyoto 601 (JP); Takama, Toshio, c/o Kyoto Dai-ichi Kagaku Co, Ltd., Kyoto 601 (JP)
(74) Representative: Schoppe, Fritz, Dipl.-Ing.

(57) **Abstract**

A positive charged gold colloid (72) is mixed into a solution containing single-stranded DNAs (70) having a specific base sequence to be detected, for forming a DNA probe (74). A solution containing a DNA specimen (76) and the DNA probe (74) is introduced into a temperature controlled cell (78), which in turn is heated to 90 to 95 _{°} C for cleaving the DNA specimen (76), and thereafter the temperature is gradually reduced for carrying out incubation at 37 to 70 _{°} C for several minutes to one hour.

If the DNA specimen (76) includes the base sequence corresponding to the single-stranded DNAs forming the DNA probe (74), single-stranded DNAs (76a) prepared from cleaved DNA specimen (76) and the DNA probe (74) are hybridized to form double-stranded DNAs in the DNA probe (74). Thus, it is possible to simply detect the specific base sequence with no requirement for an operation of labelling the DNAs with chemical substances.

## Description

### BACKGROUND OF THE INVENTION

### Field of the Invention

The present invention relates to a method of and an apparatus for detecting a specific DNA-(deoxyribonucleic acid) base sequence. Such detection of a specific DNA base sequence is employed in the fields of a clinical test, gene diagnosis, pathologic study, drug quality control and the like.

### Description of the Background Art

A specific DNA base sequence is mainly detected by Southern blotting or liquid phase hybridization. However, such a method is disadvantageously complicated in treatment due to requirement for B-F separation and binding DNA fragments into membrance. As a method of detecting a specific base sequence with no requirement for B-F separation, there has been proposed a method of employing a fluorescently labelled single-stranded DNA probe and an immobilized reagent prepared by coupling single-stranded DNAs which are complementary in base sequence with the DNA probe to immobilized carriers having a high molecular weight. After DNAs in a specimen and the DNA probe react competitively with the immobilized reagent, the specific base sequence is detected by the degree of fluorescence polarization (refer to Japanese Patent Laying-Open Gazette No. 5-123196 (1993)).

On the other hand, surface sensitized Raman spectrometry is employed as a method of measuring a molecular vibrational spectrum. This method utilizes such a phenomenon that strong Raman scattering is caused when a substance is adsorbed on a surface of an electrode or colloid of noble metal such as gold or silver (refer to "Bunseki" 1993, pp. 577 to 585, for example). Protein, bilirubin and the like are studied through such surface sensitized Raman spectrometry. It is known that a vibrational spectrum is reinforced to 10⁷ to 10⁸ times upon occurrence of surface sensitized Raman scattering, to enable measurement in high sensitivity.

However, the method of employing the fluorescently labelled DNA probe and the immobilized complementary DNAs is disadvantageously complicated in treatment and inferior in convenience due to requirement for labelling DNAs with various chemical substances.

### SUMMARY OF THE INVENTION

A first object of the present invention is to provide a method which can simply detect a specific base sequence with no requirement for an operation of labelling DNAs with chemical substances.

A second object of the present invention is to provide an apparatus for implementing the aforementioned specific base sequence detecting method.

The inventive method of detecting a DNA base sequence uses a DNA probe prepared by labelling specific single-stranded DNAs having a characteristic base sequence of target DNAs to be detected with noble metal colloidal particles, and includes a step of cleaving specimen DNAs to single-stranded DNAs by temperature increase, a step of mixing the DNA probe with the DNA specimen before or after the cleaving step, a hybridization step of reducing the temperature of a mixed solution of the cleaved DNA specimen and the DNA probe for bonding the DNA probe with the cleaved specimen DNAs which are complementary in base sequence therewith, and a step of thereafter measuring Raman scattering light generated by irradiating the mixed solution with excitation light. The DNA probe is prepared by bonding the specific single-stranded DNAs with the noble metal colloidal particles of gold, silver or copper. The colloidal particles are properly 0.8 to 200 nm in particle diameter.

A preferable mode of detecting the base sequence corresponding to the DNA probe includes a method of spectrometrically analyzing Raman scattering light components obtained from the DNA probe before and after the hybridization step and detecting the base sequence from the existance of the difference between the characteristics of the spectra of the Raman scattering light components.

While application of the present invention to a heterogeneous detecting method of carrying out B-F separation is not excluded, Raman scattering light can be preferably measured with no B-F separation after the hybridization step.

The inventive apparatus for detecting a DNA base sequence comprises a cell having a temperature sensor and heating/cooling means, which can store the mixed solution of the DNA specimen and the DNA probe, a light source part for irradiating the mixed solution stored in the cell with excitation light, a spectrometric part for spectrometrically measuring Raman scattering light components from the mixed solution stored in the cell, and a temperature controller for changing the temperature of the cell in accordance with a predetermined program through the temperature sensor and the heating/cooling means of the cell.

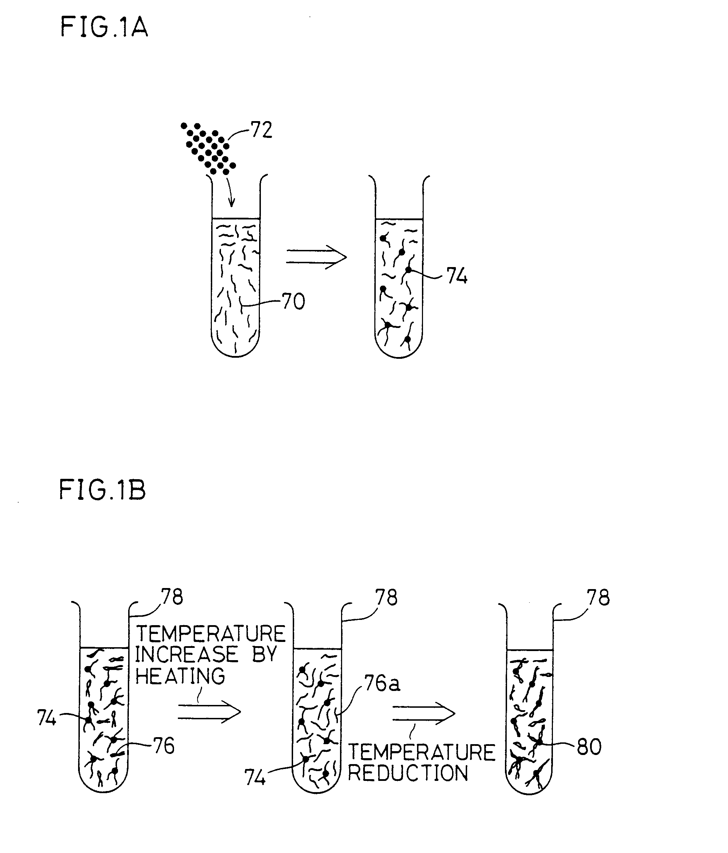

Fig. 1A illustrates a process of forming DNA probe 74 by mixing a positive charged gold colloid 72 into a solution containing specific single-stranded DNAs 70 having a characteristic base sequence of target DNAs to be detected.

Fig. 1B illustrates a method of detecting a base sequence corresponding to the single-stranded DNAs forming the DNA probe 74. A solution containig a DNA specimen 76 for measurement is introduced into a temperature controlled cell 78 and the solution is adjusted to pH 4 to 8.5 by a tris- hydrochloric acid buffer or a phosphoric acid buffer, thereafter the DNA probe 74 is mixed with the solution. The temperature controlled cell 78 is heated to a temperature of 90 to 95 °C, for cleaving the DNA specimen 76 and converting the same to single-stranded DNAs 76a. Thereafter the temperature is gradually reduced for carrying out incubation at 37 to 70 _{°} C for several minutes to one hour. If the DNA specimen 76 includes a base sequence corresponding to the single-stranded DNAs forming the DNA probe 74, the corresponding single-stranded DNAs 76a of the DNA specimen 76 and the DNA probe 74 are hybridized to form double-stranded DNAs in the DNA probe 76. Numeral 80 denotes the DNA probe provided with the double-stranded DNAs.

Alternatively, the DNA probe 74 may be mixed with the DNA specimen 76 after the DNA specimen 76 is cleaved into the single-stranded DNAs 76a.

In order to detect whether or not DNAs of a specific base sequence corresponding to the single-stranded DNAs of the DNA probe is present in the DNA specimen, the solution containing the DNA probe in advance the hybridization step and the solution after the hybridization step are irradiated with excitation light for spectrometrically analyzing Raman scattering light components from the respective solutions. The Raman scattering light components obtained from the double-stranded DNA substance are reinforced by presence of the noble metal colloidal particles on which the DNA substance is coupled. When double-stranded DNAs are formed by the hybridization, different Raman spectra are obtained before and after the hybridization.

The Raman scattering light components supply information as to molecular vibrational spectra, which sensitively depend on steric structures and electronic states of the molecules. The feature thereof resides in that a Raman frequency which is decided by energy change in molecular vibration corresponds to a difference between two electronic vibrational energy levels forming the substance. A vibrational spectrum obtained from Raman scattering light, which is sensitive to individualities of the molecules, provides effective means of high accuracy for identifying the molecules by means such as the theory of reference vibrational analysis of the molecules and analytical treatment. Therefore, it is also possible to presume molecular structures and measure concentration from the molecular spectrum.

The molecular structures are different between the DNA probes bonded with the noble metal colloid, which are in the state of single-stranded DNAs and in the state of double-stranded DNAs converted by hybridization with the single-stranded specimen DNAs having the complementary base sequence, and hence Raman spectra thereof are also different from each other.

When the double-stranded DNAs are formed in the DNA probe by hybridization, detection sensitivity may also be different.

When double-stranded DNAs are thus formed in the DNA probe, it is possible to detect that the DNA specimen contains the DNAs having the specific base sequence from change in peak positions of the Raman spectra, or further from change in detection sensitivity.

According to the present invention, the DNA probe is prepared by labelling single-stranded DNAs having the specific base sequence to be detected with noble metal colloidal particles and mixed with single-stranded DNAs prepared by heating to cleave specimen DNAs, and the temperature is reduced to attain conditions for causing hybridization. The DNA probe is irradiated with excitation light before and after the hybridization step for measuring Raman scattering light components, thereby detecting whether or not the DNA specimen contains DNAs which are complementary in base sequence with the DNAs forming the DNA probe. Thus, it is possible to simply detect the specific base sequence in a short time.

According to the inventive method, it is possible to measure the Raman scattering light components with no requirement for B-F separation, whereby the treatment is simplified.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1A illustrates a step of forming a DNA probe;
Fig. 1B illustrates a hybridization step;
Fig. 2 is a block diagram schematically showing an optical system for carrying out the method according to the present invention;
Fig. 3 is a block diagram showing an optical system according to an embodiment of the present invention in detail;
Fig. 4 is a vertical sectional view showing an example of a preferable cell;
Fig. 5 is a block diagram showing a temperature control system for the cell shown in Fig. 4; and
Fig. 6 is a partially fragmented vertical sectional view showing another example of a preferable cell.

### DESCRIPTION OF THE PREFERRED EMBODIMENTS

It is possible to electrostatically and hydrophobically bond negatively charged single-stranded DNAs with positively charged colloidal particles. A positively charged gold colloid is commercially available in the trade name of "GENOGOLD" (by BioCell, U.S.A.), for example. When single-stranded DNAs are mixed with such a positive charge gold colloid, it is possible to readily obtain the DNA probe in which the gold colloid is bonded with the single-stranded DNAs. Unreacted single-stranded DNAs which are not bonded with the colloidal particles are removed by ultracen- trifugation, gel filtration or high performance liquid chromatography. In order to inhibit DNAs other than the target ones which are present in a specimen from non-specific adsorption to the gold colloid, the DNA probe is preferably blocked with a protein solution of BSA or casein, or a protein solution having an isoelectric point which is lower than the pH value of a reaction solution containig the DNA probe.

Single-stranded DNAs may alternatively be bonded with colloidal particles by maleimide-reacting single-stranded DNAs into which SH groups are introduced with colloidal particles (by Nano Probe, U.S.A.) bonded with maleimide groups for making covalent bonding.

It is also possible to bond single-stranded DNAs with colloidal particles by avidin-biotin bonding single-stranded DNAs into which biotin is introduced with streptoavidin-bonded colloidal particles (by Nano Probe, Zaimet(U.S.A.), or E.Y Laboratory-(U.S.A.)).

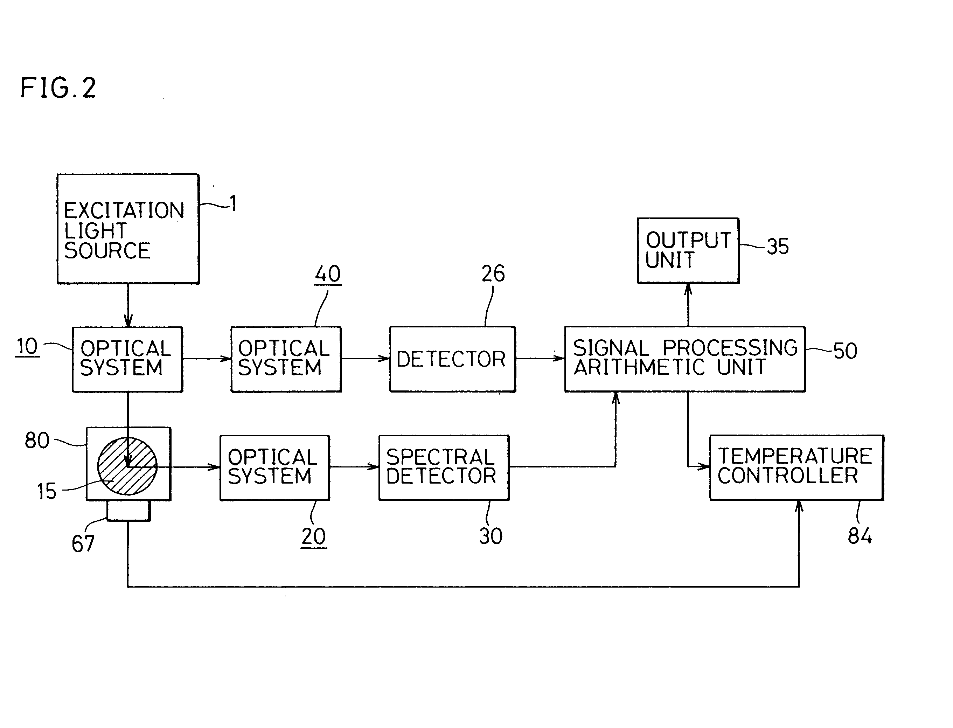

Fig. 2 shows an DNA detector according to an embodiment of the present invention.

A temperature controlled cell 80 comprising a heater and a Peltier element stores a sample 15 containing DNA specimen and DNA probe having a specific DNA base sequence to be detected. Numeral 67 denotes a temperature sensor for detecting the temperature of the cell 80. A detection signal from the temperature sensor 67 is input in a temperature controller 84, which in turn increases the temperature of the cell 80 to a level necessary for cleaving the DNA specimen and thereafter reduces the temperature to a level for hybridization.

Numeral 1 denotes an excitation light source, which is formed by a laser unit, for measuring Raman scattering light. The laser unit can be selected from lasers having wide ranges of wavelengths over near-ultraviolet to near-infrared regions such as a continuously oscillating Ar ion laser, a Kr ion laser, an He-Ne laser and an He-Cd laser, and a pulse laser such as an Nd:YAG laser. When spontaneous emission light of the laser unit is screened so that only an oscillation beam is utilized as the excitation light, the laser unit may be combined with an interference filter or a spectroscope. Alternatively, the spontaneous emission light may also be simultaneously applied to carry out wavelength calibration of a spectrum.

The excitation light generated from the light source 1 is separated by an optical system 10 into a measuring beam and a reference beam, so that the measuring beam is adjusted by the optical system 10 and applied to the sample 15 which is stored in the cell 80. Raman scattering light generated from the sample 15 is taken out in a direction which is at an angle of 90 _{°} with respect to the direction of incidence of the measuring beam, and detected by a spectral detector 30 including a spectroscope through an optical system 20 for adjusting the luminous flux.

On the other hand, the reference beam is detected by a detector 26 through an optical system 40 for luminous flux adjustment, in order to correct fluctuation of excitation light intensity. A signal processing arithmetic unit 50 corrects the Raman scattering light which is detected by the spectral detector 30 with an output of the detector 26 indicating light source intensity to obtain a Raman spectrum, thereby detecting specific base sequence. A signal processing arithmetic unit 50 controls the temperature controller 84 in accordance with a predetermined temperature program, to change the temperature of the cell 80. Numeral 35 denotes an output unit such as a printer or a CRT.

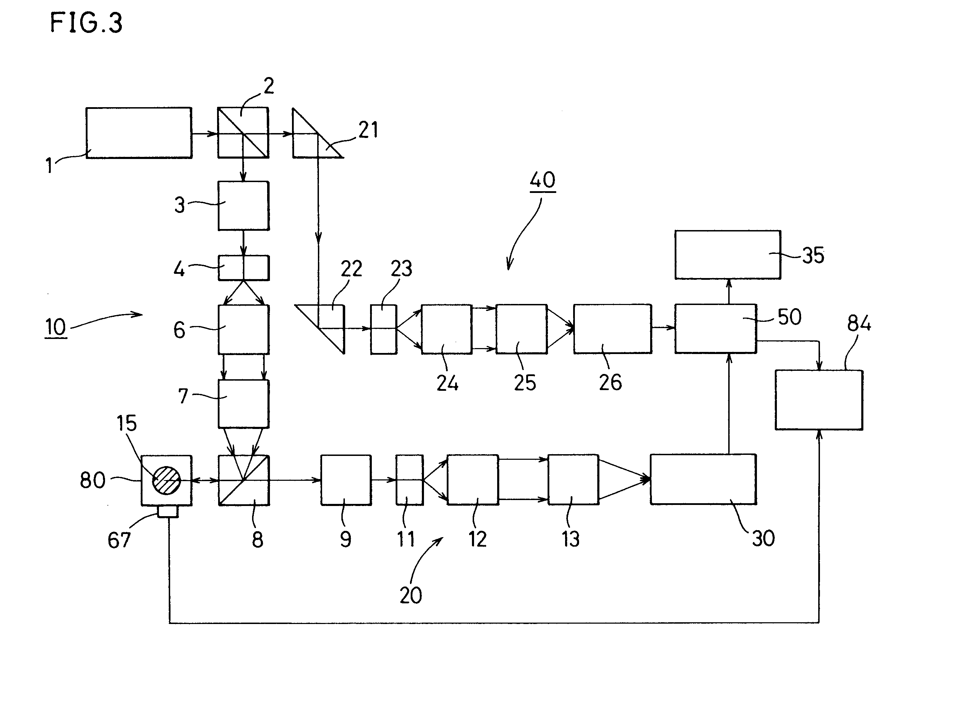

Fig. 3 shows optical systems of the DNA detector in detail. A beam splitter 2 for separating the luminous flux of the excitation light which is received from the laser light source 1 into the measuring beam and the reference beam, a convex lens 3 for condensing the measuring beam which is separated by the beam splitter 2, convex lenses 6 and 7 for adjusting the luminous flux, and a beam splitter 8 for reflecting to guide the measuring beam to the sample 15 which is stored in the cell 80 are arranged along the optical path of the measuring beam, as the optical system 10 for irradiating the sample 15 with the luminous flux serving as the measuring beam. On the optical path of the measuring beam, further, a filter 4 is provided between the convex lenses 3 and 6 for selecting a laser beam of one wavelength from a plurality of oscillation beams received from the laser light source 1.

On the other hand, a convex lens 9 for condensing the Raman scattering light and the measuring beam transmitted through the beam splitter 8 and convex lenses 12 and 13 for adjusting the luminous flux are arranged along the optical path of the Raman scattering beam which is generated from the sample 15 stored in the cell 80, as the optical system 20 for extracting the Raman scattering light in a direction which is at an angle of 180 with respect to the direction of incidence of the measuring beam. A filter 11 for removing an excitation light component is provided between the convex lenses 9 and 12.

On a reference beam side, further, reflecting mirrors 21 and 22 for bending the optical path and convex lenses 24 and 25 for adjusting the luminous flux are arranged along the optical path, in order to guide the reference beam to the detector 26. A filter 23 of the same wavelength characteristics as the filter 4 provided on the measuring beam side is provided between the reflecting mirror 22 and the convex lens 24.

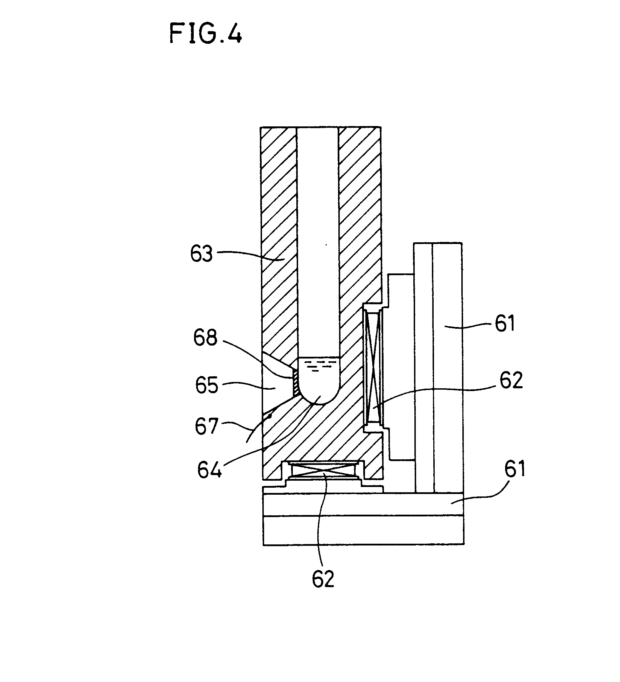

Fig. 4 shows an exemplary temperature controlled cell 80.

Referring to Fig. 4, numeral 63 denotes an aluminum cell, comprising an aluminum metal block having a hole which is provided in its upper end toward its interior, for storing a sample. An inner surface of this hole is mirror-polished, and subjected to gold plating to be increased in infrared reflectance. A window 65 is provided in a bottom portion of the hole for applying excitation light to a sample 64 from the exterior and taking out Raman scattering light from the sample 64 toward the exterior, while a quartz glass window plate 68 is fitted in this window 65. In the metal block forming the cell 63, heating/cooling devices 62, 62 including heaters and Peltier elements are provided on side and bottom portions of the hole respectively, while diffusion plates 61, 61 of aluminum are in contact with other sides of the heating/cooling devices 62, 62. A temperature sensor 67 is embedded in the metal block forming the cell 63. In the cell 63 shown in Fig. 4, the direction of incidence of excitation light is at 180 with respect to the direction for taking out the Raman scattering light.

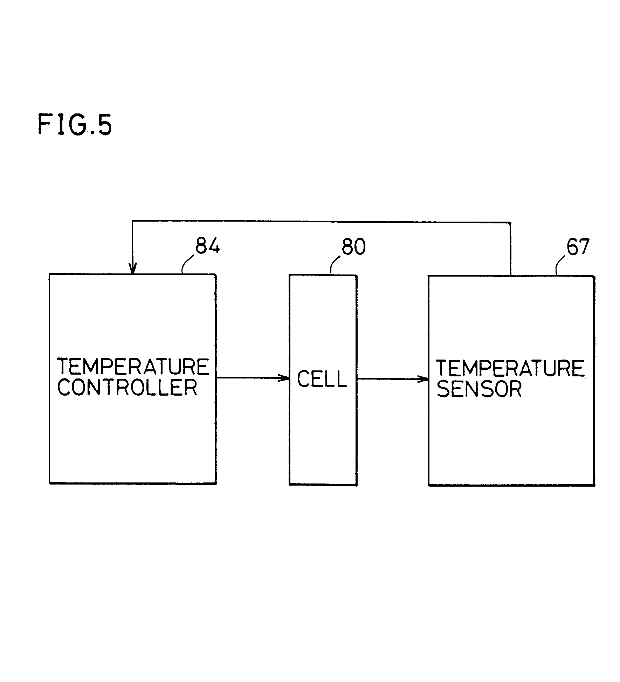

In order to increase and reduce the temperature of the cell 63, a temperature controller 84 is provided as shown in Fig. 5, for incorporating a detection signal of the temperature sensor 67 and controlling the amounts of currents to be fed to the heating/cooling devices 62, 62.

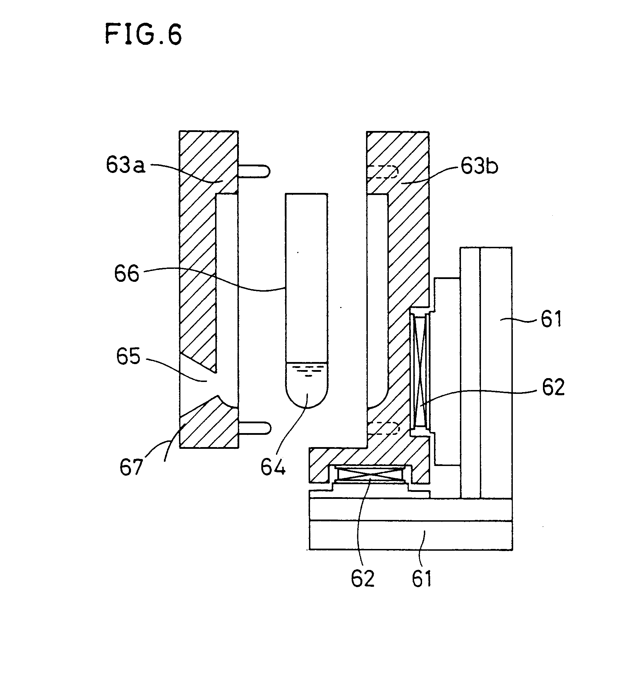

Fig. 6 shows another exemplary cell. As compared with the cell 63 shown in Fig. 4 which is integrally formed by hollowing the metal block, a quartz glass cell 66 is employed in Fig. 6. An aluminum metal block is divided into two portions 63a and 63b, while cavities for storing the cell 66 are formed in opposite parts of these portions 63a and 63b, so that the portions 63a and 63b of the metal block are combined with each other to enclose the cell 66 with the cavities. The cell 66 is in contact with the metal block formed by the portions 63a and 63b which are combined with each other. A window 65 is formed in a position of the metal block portion 63a corresponding to a bottom portion of the cell 66, in order to apply excitation light and to take out Raman scattering light. Heating/cooling devices 62, 62 for controlling the temperature are so mounted that each one side thereof is in contact with the metal block portion 63b, while diffusion plates 61, 61 are provided on other sides of the heating/cooling devices 62, 62 and a temperature sensor 67 is embedded in the metal block poriton 63a.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A method of detecting a DNA base sequence characterized by comprising:
a step of cleaving specimen DNAs(76) to single-stranded DNAs(76a) by temperature increase;
a step of mixing a DNA probe(74) with said specimen DNAs(76) before or after said cleaving step, said DNA probe(74) being prepared by labelling specific single-stranded DNAs(70) having a characteristic base sequence of target DNAs to be detected with noble metal colloidal particles(72);
a hybridization step of reducing the temperature of a mixed solution of said cleaved specimen DNAs(76a) and said DNA probe(74) for bonding said DNA probe(74) with the cleaved specimen DNAs(76a) being complementary in base sequence therewith; and
a step of measuring Raman scattering light generated by irradiating said mixed solution with excitation light, thereby detecting said base sequence corresponding to said DNA probe(74).

2. A method of detecting a DNA base sequence in accordance with claim 1, being a homogeneous detecting method of irradiating said mixed solution with said excitation light while not carrying out B-F separation after said hybridization step.

3. A method of detecting a DNA base sequence in accordance with claim 1, wherein said detection of said base sequence corresponding to said DNA(74) probe is carried out through difference between characteristics of spectra of Raman scattering light components being obtained by spectrometrically analyzing said DNA probe(74) before and after said hybridization step.

4. A method of detecting a DNA base sequence in accordance with claim 1, wherein a noble metal colloid of said colloidal particles is prepared from any metal selected from gold, silver and copper, said colloid being 0.8 to 200 nm in particle diameter.

5. An apparatus for detecting a DNA base sequence characterized by comprising:
a cell(80), having a temperature sensor(67) and electronic heating/cooling means(62), being capable of storing a mixed solution of a DNA specimen and a DNA probe prepared by labelling single-stranded DNAs having a specific base sequence to be detected with noble metal colloidal particles;
a light source part(1) for irradiating said mixed solution stored in said cell(80) with excitation light;
a spectrometric part(30) for spectrometrically measuring Raman scattering light from said mixed solution being stored in said cell(80); and
a temperature controller(84) for changing the temperature of said cell(80) in accordance with a predetermined program through said temperature sensor(67) and said heating/cooling means(62) of said cell(80).

6. An apparatus for detecting a DNA base sequence in accordance with claim 5, wherein said light source(1) part comprises a laser unit.

7. An apparatus for detecting a DNA base sequence in accordance with claim 6, wherein said light source part further comprises spectral means for selecting an oscillation beam of a specific wavelength from light being generated from said laser unit.

8. An apparatus for detecting a DNA base sequence in accordance with claim 5, further comprising:
an optical system(10) for separating said excitation light from said light source part(1) into a measuring beam and a reference beam, and
correction means(50) for correcting an output of said spectrometric part(30) through said measuring beam by intensity of said reference beam.

9. An apparatus for detecting a DNA base sequence in accordance with claim 5, wherein said cell(80) is formed by a heat conductive metal block(63) having a hole provided in its upper end toward its interior for storing said mixed solution, said hole being provided with a mirror-polished inner surface being subjected to gold plating, and a bottom portion having a window(68) for receiving said excitation light and emitting said Raman scattering light with a quartz glass window plate being fitted therein.

10. An apparatus for detecting a DNA base sequence in accordance with claim 5, wherein said sample cell consists of:
a quartz glass cell(66), and
a heat conductive metal block having a hole in its upper end toward its interior for storing said quartz glass cell(66), said hole being provided in its bottom portion with a window(65) for receiving said excitation light and emitting said Raman scattering light,
said electronic cooling/heating means(62) and said temperature sensor(67) being provided on said metal block.

11. An apparatus for detecting a DNA base sequence in accordance with claim 10, wherein said metal block consists of two portions-(63a,63b) being combined with each other for defining said hole for storing said quartz glass cell(66).
